Europäisches Patentamt

European Patent Office

Office européen des brevets

(11) Publication number: **0 294 805**
**A2**

# (12) EUROPEAN PATENT APPLICATION

(21) Application number: 88109203.5

(22) Date of filing: 09.06.88

(51) Int. Cl.4: **C12N 15/00 , C12N 1/18 ,**
**//(C12N1/18,C12R1:865)**

The applicant has filed a statement in accordance with Rule 28 (4) EPC (issue of a sample only to an expert). Accession number(s) of the deposit(s): FERM P-9065.

(30) Priority: 12.06.87 JP 145316/87

(43) Date of publication of application:
14.12.88 Bulletin 88/50

(84) Designated Contracting States:
AT BE CH DE ES FR GB GR IT LI LU NL SE

(71) Applicant: **Eisai Co., Ltd.**
**6-10, Koishikawa 4-chome Bunkyo-ku**
**Tokyo 112(JP)**

(72) Inventor: **Nagasu, Takeshi**
**Kijo heights 17, 16-23 Chuo 2-chome**
**Tsuchiura-shi Ibaraki(JP)**
Inventor: **Iwama, Kiyoshi**
**530-188 Karasuyama**
**Tsuchiura-shi Ibaraki(JP)**
Inventor: **Ikeda, Yasunori**
**28-9, Kariyacho 5-chome**
**Ushiku-shi Ibaraki(JP)**

(74) Representative: **Hansen, Bernd, Dr.rer.nat. et al**
**Hoffmann, Eitle & Partner Patentanwälte**
**Arabellastrasse 4**
**D-8000 München 81(DE)**

(54) Temperature-sensitive sugar chain mutant.

(57) A temerature-sensitive sugar chain mutant is obtained by mutating a parent strain belonging to Sac-charomyces cerevisiae through a $^3$H-mannose suicide method, characterized in that said mutant is transformed with a manifestation vector including an inducible promotor to be used as a host component in a host/vector system, after said mutant is incubated at a low temperature and subsequently at a high temperature under conditions suitable for the employed vector to thereby induce the manifestation of the aimed product.

## Temperature-sensitive Sugar Chain Mutant

### (1) Field of the Invention

This invention relates to a temperature-sensitive sugar chain mutant which is prepared in order to give a product carrying high-mannose core type sugar chains and to facilitate the secretion of said product, when used as a host component in a host/vector system.

### (2) Prior Art

When a protein is manifested by using a yeast as a host in gene recombination, sugar chains would attach to a so-called asparagine triplet. The attachment of excessive sugar chains might sometimes lower the activity of the product (see ref. 1, 2 and 3).

1) Robert E. Cohen, Clinton E. Ballou, et. al.: The Journal of Biological Chemistry, Vol 255, No. 16, pp7700-7707 (1980).

2) Pei-Kuo Tsai, Clinton E. Ballou, et. al.: ibid. Vol 259, No. 6, pp3805-3811 (1984).

3) Tim C. Huffaker, Phillips W. Robbins: ibid. Vol 257, No. 6, pp3203-3210 (1982).

However it has not been sufficiently clarified yet which conditions would cause the abovementioned attachment of excessive sugar chains. Therefore neither practical method nor appropriate host has been proposed so far for satisfactorily avoiding the attachment of excessive sugar chains. Since the attachment of excessive sugar chains is a physiological phenomenon which aims at the maintenance of the survival of the yeast and the acceleration of the secretion of the product, it is extremely difficult to avoid the same while satisfying these requirements to a certain extent.

In general, a product obtained through the manifestation with a yeast carries a sugar chain having a high-mannose core, characteristic to the yeast, to which two or more outer mannose chains are bound. Thus this product fails to fully exert its activity. The references 1, 2 and 3 cited above have disclosed each a process for solving this problem by blocking the addition of outer mannose sugar chains midway in the secretion pathway to thereby give a product still carrying a sugar chain of high-mannose core type and a mutant sec 18 has been developed therefor. In this process, however, the secretion of a product per se, which is an important factor of gene recombination, is blocked, which considerably restricts the practical application thereof.

Thus the prior art fails to simultaneously satisfy the requirement for inhibiting the attachment of excessive sugar chains and that for accelerating the secretion of a product.

### (3) Summary of the Invention

Under these circumstances, the present inventors have studied how to avoid the attachment of excessive sugar chains by incubating a mutant, which is obtained through temperature-sensitive mutation of a gene which controls the attachment of outer chains without affecting the secretion pathway, under specified conditions. As a result, they have found that this object can be achieved by employing a mutant obtained by mutating Saccharomyces cerevisiae through the $^3$H-mannose suicide method as a host, incubating the hose at a low temperature to thereby give a sufficient amount of cells, then incubating the cells at a high temperature, and inducing the manifestation of the aimed product, thus completing the present invention. Accordingly, it is the gist of the present invention to provide a temperature sensitive sugar chain mutant characterized by being treated in the abovementioned manner.

Thus, the invention comprises a temperature-sensitive sugar chain mutant of Saccharomyces cerevisiae which

(1) is capable of expressing a protein having a sugar chain including an inner high-mannose core but without an outer mannose chain, when (a) the said mutant is transformed as a host with an expression vector for the protein including an inducible promotor, (b) the transformed mutant is cultivated at first at a low temperature under conditions not suitable for expressing the protein and (c) the transformed mutant is subsequently cultivated at a high temperature under conditions suitable for inducing the expression of the desired protein, and

(2) is obtained by a $^3$M-mannose suicide method of a S. cerevisiae parent strain.

Further, the invention concerns a method of obtaining said temperature-sensitive sugar mutant as characterized in claims 3 to 6. Moreover, the invention encompasses a process for producing a protein which has a sugar chain including an inner high-mannose core but without an outer mannose chain as characterized in claims 7 and 8.

The invention concerns also a transformant obtained by transforming the mutant as defined in claim 1 with an expresion vector for a protein including an inducible promotor.

Now the present invention will be described in detail.

The term "temperature-sensitive mutation" as used herein is defined as follows. Namely, when a specified gene showing a substantially normal phenotype at a low temperature shows a mutated phenotype under high temperature conditions, this mutation is called the temperature-sensitive mutation. When the mutation relates to sugar chain(s), it is called the temperature-sensitive sugar chain mutation. In the present invention, particular attention is to be paid to the extent of the attachment of sugar chains to a product. That is to say, the mutant of the present invention shows a normal phenotype allowing sugar chain attachment widely ranging from a mannose core to outer chain attachment thereto at a low temperature, while it shows a mutated phenotype wherein the attachment of outer sugar chains is not allowed and thus a sugar chain remains of a mannose core type at a high temperature.

The term "induction" as used herein means that a promotor which would exert no effect under certain conditions is activated by appropriately altering the incubation conditions and thus a gene coding for the product begins to manifestate the product thereby. The above promotor causing the induction is called an inducible promotor.

Any yeast belonging to Saccharomyces cerevisiae may be employed in the present invention. Alternately those obtained through genetic mating of two or more yeast may be used. An example thereof is EHA-1C obtained by genetic mating of three yeasts X-2180-1A, DBY 746 and 20B-12. According to the catalogue of Yeast Genetic Stock Center (see ref. 4), the X-2180-1A, DBY 746 and 20B-12 show genotypes of aSUC2 mal mel gal2 CUP1, a his3-Δ1 leu2-3 leu2-112 ura3-52 trp1-289a and a pep4-3 trp1, respectively.


(4) Catalogue Yeast Genetic Stock Center, fifth Ed. (04/01/84), pp4, pp12, pp30)


Thus the aimed EHA-1C of a genotype of a leu2 pep4 may be obtained by first mating X-2180-1A with DBY 746 to thereby give DX 4b of a genotype of a leu2-3, 112 and then mating the Dx 4b with 20B-12. Since EAH-1C includes leu2 as a gene marker, it is available as a host for a manifestation vector carrying LEU2 gene (normal). The $^3$H-mannose suicide method is widely known and may be carried out according to the description of reference 5.

5) Tim C. Huffaker, Phillips W. Robbins: Proc. Natl. Acad. Sci. USA, Vol 80, pp7466-7470 (1984).

It comprises incubating yeast cells treated with NaNO$_2$ in a $^3$H-mannose-containing medium. As the result of the NaNO$_2$ treatment, cells forming outer mannose chains would incorporate a large amount of $^3$H-mannose and thus die from the radiation. In contrast thereto, mutated cells carrying shortened sugar chains would incorporate a smaller amount of $^3$H-mannose. Thus the latter cells survivie and are gradually enriched, which makes the collection thereof possible. In the present invention, it is further necessary that the aimed mutant is temperature-sensitive. Thus the $^3$H-mannose suicide method is carried out at a high temperature. Namely, yeast cells preliminarily treated with NaNO$_2$ are incubated in a $^3$H-mannose-containing medium at a high temperature. Then cells showing a decrease in the incorporation of $^3$H-mannose are concentrated. Further those which are temperature-sensitive with regard to multiplication are selected in the secondary screening. Among the selected cells, those producing invertase having no outer mannose chain at a high temperature are selected by invertase staining and then purified. A YPD agar medium is preferable as the medium to be used in the $^3$H-mannose suicide method, though it is not restricted thereby.

In the present invention, the terms "high temperature" and "low temperature" are used relative to each other. Namely, they mean two temperatures at which a yeast can grow and the former is generally higher than the latter by 5 to 15 °C. For example, a low temperature means 20 to 30 °C while a high temperature means 35 to 40 °C, though they are not restricted thereby.

The temperature-sensitive sugar chain mutant Saccharomyces cerevisiae EHF 2C according to the present invention can be obtained by treating the abovementioned strain EHA-1C by the $^3$H-mannose suicide method, as will be shown in Example hereinafter. This mutant has been deposited with the Fermentation Research Institute as FERM P-9065.

When the mutant of the present invention is to be used as a host component in a host/vector system, a manifestation vector including an inducible promotor as defined above is prepared before the mutant is transformed by said vector according to, for example, the description of reference 6.


(6) T. Maniatis, et. al.: Molecular Cloning, Cold Spring Harbor Laboratory (1982).


The transformant thus prepared is first incubated at a low temperature as defined above in, for example, a YPD medium until a sufficient amount of cells are obtained. This incubation is to be carried out under such conditions as not to induce the manifestation of the aimed product, which are preliminarily determined depending on the employed promotor. Subsequently the cells are incubated at a high temperature as defined above under such conditions as to induce the manifestation of the product. The conditions for inducing the manifesta tion of the product are to be determined depending on each promotor. Since this transformant can not attach outer chains at a high temperature, every sugar chain of the obtained product remains of the mannose core type. Different from the description of references 1 to 3, the mutant of the present invention does not suffer from blocking in the secretion pathway. Thus it can readily secrete the product similar to usual yeasts.

Brief Description of the Drawings:

Fig. 1 is a graph showing the change in multiplication with the lapse of time.
Fig. 2 is a schematic figure showing the results of electrophoresis.

Example:

To further illustrate the present invention, the following Example will be given.

Example 1

1) Preparation of EHA 1C:

Mating of X-2180-1A (genotype: a SUC2 mal mel gal2 CUP1), DBY 746 (genotype: a, his3 Δ1 leu2-3, leu2-112, ura3-52 trp1-289a) and 20B-12 (genotype: a pep4-3 trp1) each obtained from Yeast Genetic Stock Center was carried out (see ref. 7)

7) Methods in yeast genetics, Cold Spring Harbor Laboratory).

First, the DBY 746 was mated with the X-2180-1A followed by sporulation and tetrad analysis. After analyzing the phenotypes of the mating types leu and trp, a strain DX 4B requiring leu alone and showing a genotype of mating type a was obtained. Subsequently the obtained DX 4B (a leu) was mated with the 20B-12 (a pep4-3 trp1) followed by sporulation and tetrad analysis. After analyzing the phenotypes of mating types leu, trp and pep, a strain EHA 1C showing a genotype of a leu, pep was obtained.

2) Incorporation of $^3$H-mannose:

The EHA 1C treated with $NaNO_2$ was incubated in a YPD medium at 25° C. 20 hours thereafter, the incubation temperature was raised to 36° C. After incubating the strain at this temperature for one hour, 4.5 ml of the incubation medium was centrifuged. To the cells thus obtained, 100 $\mu l$ of a YPD medium containing 0.1 % of glucose and 1.4 mCi of $^3$H-mannose was added and the cells were cultured at 36° C for one hour. Then 5 ml of cold distilled water was added thereto and the resulting mixture was washed with water thrice. The cells were collected by centrifugation and a mixture of 25 % glycerol with 50 % YPD medium was added thereto. The mixture was stored at -80° C. After two, three and four weeks, the cells were taken out and inoculated into a YPD medium plate to thereby form colonies.

3) Confirmation of the incorporation of $^3$H-mannose:

The plate inoculated with the temperature-sensitive strain as obtained in 2) was replicated to two YPD medium plates and the strain was incubated at 25° C overnight. Then each plate was further replicated to a membrane filter for blotting, placed on a filter containing a YPD medium and incubated at 25° C for four hours. After raising the incubation temperature to 36° C, the incubation was continued for additional one hour. Then the filter was placed on a filter containing 100 $\mu$Ci/ml of $^3$H-mannose or 2 $\mu$Ci/ml of $^{35}$S-methionine and incubated thereon at 36° C for 15 minutes to thereby label the strain. Each filter was further placed on a filter containing 5 % glutaraldehyde and fixed thereon at 36° C for 30 minutes. After washing with water and drying, the filter was placed on a tritium filter and exposed to light at room temperature for two days.

4) Preparation of sample for electrophoresis:

The strain was incubated in a YPD medium at 25° C for 20 hours and then at 37° C for 1.5 hour. Then cells of a medium showing an $OD_{600}$ value of 20 were collected and washed with water. 5 ml of a YPD medium containing 0.05 % of glucose was added thereto and the cells were incubated at 37° C for 45 minutes. Then 5 ml of 20 mM sodium nitride cooled to 0° C was added thereto and the cells were collected and washed with water. 50 $\mu$l of a TBP buffer and glass beads were added to the cells and the mixture was treated with a vortex stirrer at 4°C for 10 minutes. The mixture containing the cells thus ground was centrifuged. To 50 $\mu$l of the supernatant, 25 $\mu$l portions of 5 % SDS, 50 % glycerol and 0.1 % Bromphenol Blue were added and the mixture was allowed to stand at room temperature for eight hours or longer. Thus a sample for electrophoresis was obtained.

5) Electrophoresis and active invertase activity staining:

SDS (0.1 %) polyacrylamide gel electrophoresis was carried out. 5 % acrylamide was employed as a separation gel while 3 % acrylamide was employed as a stacking gel. After the completion of the electrophoresis, the gels were shaken in 2 % TR-X-100 for one hour to thereby remove the SDS. After washing with water, the gels were immersed in a 0.1 M sucrose/0.1 M acetate buffer (pH 5.1) and incubated at 37°C for 20 minutes. After washing with water, they were immersed in 0.1 % TTC/0.5 N NaOH and heated on a water bath to thereby turn the invertase red.

6) Results:

120,000 strains were isolated in total from those obtained two, three and four weeks after effecting the $^3$H-mannose suicide method as described in 2). Among these strains, 874 temperature-sensitive ones were selected. 64 strains, each incorporated $^{35}$S-methionine and little $^3$H-mannose during the procedure as shown in 3), were further selected therefrom.

These strains were incubated at a high temperature. Then mutants whose electrophoretic patterns showed almost exclusively low-molecular weight invertase and scarcely any high-molecular weight one, i.e., those carrying mannose core type sugar chains were selected. Thus the aimed strain EHF 2C was obtained therefrom.

Experimental Example 1

The mutant EHF 2C as obtained in Example 1 was inoculated into a YPD medium containing 1 % of yeast extract, 2 % of bactopeptone and 2 % of glucose and incubated in three steps at 25, 30 and 37°C. During the incubation period, a change in the multiplication with the lapse of time was monitored by determining the turbidity of the medium.

The wild strain EHA 1C and mutant EHF 2C as described in Example 1 were inoculated each into a YPD medium. Each strain was incubated in three steps at 25, 30 and 37°C for six hours. The invertase thus obtained was subjected to electrophoresis and invertase activity staining according to 4) and 5) in Example 1.

Results:

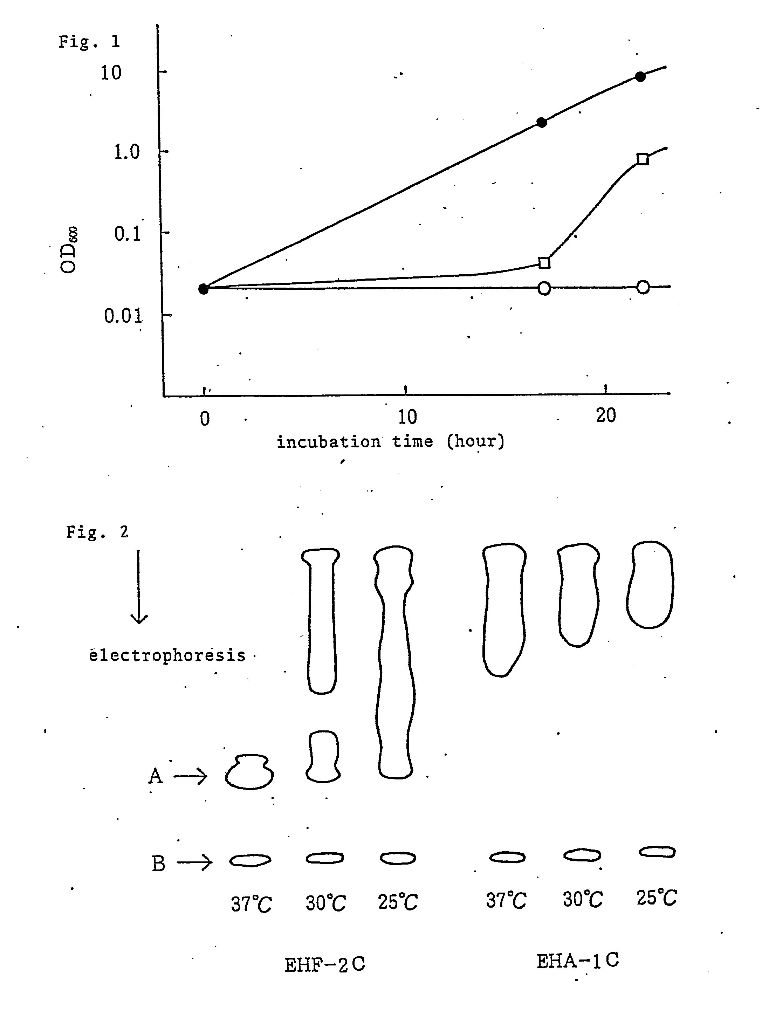

Figs. 1 and 2 show the results.

Fig. 1 is a graph showing the change in multiplication with the lapse of time, wherein the ordinate refers to turbidity (OD$_{600}$). In this graph, -●-●-, -□-□- and -O-O- represent the results at 25, 30 and 37°C, respectively.

Fig. 2 shows the results of the electrophoresis of the invertase, wherein the ordinate refers to the migration distance. The left-half ones show the data of EHF 2C at the temperatures as defined above, while the right-half ones show those of EHA 1C. The arrows A and B indicate the positions of development of core chain invertase and cytoplasmic invertase, respectively.

Fig. 1 indicates that the mutant of the present invention multiplied exclusively at a low temperature. Fig. 2 indicates that both of the wild strain and mutant actively secreted the products out of the cells; and that the product of the mutant carried sugar chains remaining of a core type, in particular completely at a high temperature, differing the product of the wild strain which showed excess sugar chains caused by the attachment of outer chains.

These results obviously suggest the following facts. When the mutant of the present invention is to be used as a host component in a host/vector system, it is first incubated at a low temperature and then at a high temperature under conditions suitable for the employed promotor to thereby induce the manifestation of the aimed product. It has been found that the product thus obtained carries a short sugar chain of core type and is very readily secreted.

**Claims**

1. A temperature-sensitive sugar chain mutant of <u>Saccharomyces cerevisiae</u> which

(1) is capable of expressing a protein having a sugar chain including an inner high-mannose core but without an outer mannose chain, when (a) the said mutant is transformed as a host with an expression vector for the protein including an inducible promotor, (b) the transformed mutant is cultivated at first at a low temperature under conditions not suitable for expressing the protein and (c) the transformed mutant is subsequently cultivated at a high temperature under conditions suitable for inducing the expression of the desired protein, and

(2) is obtained by a $^3$M-mannose suicide method of a S. cerevisiae parent strain.

2. A mutant of claim 1, which is S. cerevisiae EHF2C.

3. A method of obtaining a temperature-sensitive sugar chain mutant of Saccharomyces cerevisiae as defined in claim 1, which method comprises:

(i) treating with NaNO$_2$ cells of parent yeast belonging to the genus Saccharomyces cerevisiae which produces a protein having a sugar chain including an inner high-mannose core and an outer mannose chain when the said protein is expressed, using the said parent yeast as a host in gene recombination technique,

(ii) cultivating the above-treated cells in a $^3$H-mannose-containing cultivation medium at a high temperature, thereby killing those cells which take in a large amount of $^3$H-mannose and form a protein having the outer mannose chain as well as the inner high-mannose core by virtue of radiation from $^3$H, and thus selecting mutated cells which take in a small amount of $^3$H-mannose and form a protein having only the inner-high mannose core; and

(iii) further selecting among those selected in step (ii), cells which produce invertase having no outer mannose chain at a high temperature by an invertase staining method.

4. A method of claim 3 wherein the parent yeast is S. cerevisiae EHA-IC.

5. A method of claim 3 wherein YDD medium is used as the cultivation medium.

6. A method of claim 3, 4 or 5, wherein the high temperature is 35 to 40° and the low temperature is 20 to 30°C while the difference is 5 to 15°C.

7. A process for producing a protein which has a sugar chain including an inner high-mannose core but without an outer mannose chain, which process comprises:

(a) transforming the mutant as defined in claim 1 with an expression vector for the protein incuding an inducible promotor,

(b) cultivating the transformed mutant at first at a low temperature under conditions not suitable for inducing the expression of the desired protein, thereby growing the number of the transformed mutant cells without expression of the protein, and

(c) subsequently cultivating the transformed mutant at a high temperature under conditions suitable for inducing the expression of the desired protein, thereby producing the desired protein.

8. A process of claim 7 wherein the mutant prior to the transformation is S. cerevisiae EHF2C.

9. A transformant obtained by transforming the mutant as defined in claim 1 with an expression vector for a protein including an inducible promotor.

Fig. 1

Fig. 2